(19) Europäisches Patentamt
European Patent Office
Office européen des brevets

(11) EP 0 647 606 B1

(12) EUROPÄISCHE PATENTSCHRIFT

(45) Veröffentlichungstag und Bekanntmachung des Hinweises auf die Patenterteilung:
**26.03.1997 Patentblatt 1997/13**

(51) Int Cl.[6]: **C07B 37/02**, C07C 13/70, C07C 69/618, C07D 309/12, C07C 321/28

(21) Anmeldenummer: **94115753.9**

(22) Anmeldetag: **06.10.1994**

# (54) Fullerenderivate, Verfahren zur Herstellung und deren Verwendung

Derivatives of fullerene, process for their preparation and their use

Dérivées de fullerène, procédé pour leur préparation et leur utilisation

(84) Benannte Vertragsstaaten:
**BE CH DE FR GB IT LI NL**

(30) Priorität: **12.10.1993 DE 4334661**
**23.08.1994 DE 4429807**

(43) Veröffentlichungstag der Anmeldung:
**12.04.1995 Patentblatt 1995/15**

(73) Patentinhaber: **HOECHST AKTIENGESELLSCHAFT**
**65929 Frankfurt am Main (DE)**

(72) Erfinder:
- **Bestmann, Hans Jürgen Prof. Dr.**
  **D-91056 Erlangen (DE)**
- **Hadawi, Dariusch Dr.**
  **D-91052 Erlangen (DE)**
- **Röder, Thomas Dr.**
  **D-91555 Feuchtwangen (DE)**
- **Moll, Claus**
  **D-90587 Veitsbronn (DE)**

(56) Entgegenhaltungen:
**US-A- 5 171 373**

- **ANGEWANDTE CHEMIE, Bd.105, Nr.8, August 1993, WEINHEIM Seiten 1189 - 1192 A. HIRSCH 'Die chemie der Fullerene: ein Überblick'**
- **ANGEWANDTE CHEMIE. INTERNATIONAL EDITION, Bd.1, Nr.2, 1962, WEINHEIM DE Seiten 116 - 117 H. J. BESTMANN ET AL 'Reaction of Triphenylene Alkylenes with Activated Double Bounds'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 647 606 B1

## Beschreibung

Fullerene sind käfigförmige Kohlenstoffallotrope der allgemeinen Formel ($C_{20+2m}$), wobei m eine natürliche Zahl ist. Sie enthalten zwölf Fünf- sowie beliebig viele, mindestens aber zwei Sechsringe aus Kohlenstoffatomen. Obwohl diese Verbindungsklasse erst 1985 von Kroto und Smalley nachgewiesen wurde (Nature 318 (1985) 162) und Krätschmer und Huffman erstmals 1990 über die Darstellung makroskopischer Mengen an $C_{60}$ berichteten (Nature 347 (1990) 354), sind solche Verbindungen sehr schnell auf ein breites Interesse gestoßen und wurden innerhalb kürzester Zeit Gegenstand zahlreicher Forschungsarbeiten (siehe z.B. G. S. Hammond, V. J. Keck (Editors), Fullerenes, American Chemical Society, Washington DC 1992 und Accounts of Chemical Research, Märzausgabe 1992).

Da man ein hohes Potential dieser Stoffklasse, beispielsweise im Bereich der Optoelektronik und der Wirkstofforschung, erwartet, wurden bereits umfangreiche Untersuchungen zur gezielten Derivatisierung, insbesondere von $C_{60}$ und $C_{70}$, unternommen (siehe z.B. R. Taylor, D. R. M. Walton, Nature 363 (1993) 685 und A. Hirsch, Angew. Chem. 105 (1993) 11). In verschiedenen Derivatisierungsversuchen gelang es, definierte Monoaddukte an $C_{60}$ zu isolieren.

Cyclopropanderivate wurden beispielsweise durch die Umsetzung von Fullerenen in 1.3 dipolaren Cycloadditionen mit Diazomethanderivaten (siehe z.B. F. Wudl et al., Acc. Chem. Res. 25 (1992) 157 und F. Diedrich et al., Helv. Chem. Acta 76 (1993) 1231), in [2 + 1] Carbenadditionen mit nucleophilen Glycosylidencarbenen (siehe z.B. A. Vasella et al., Angew. Chem. 104 (1992) 1383) und durch Umsetzung mit stabilisierten $\alpha$-Halocarbanionen (siehe z.B. C. Bingel, Chem. Ber. 126 (1993) 1957) erhalten. Wünschenswert war es, definierte Fullerenderivate zu synthetisieren, die ein breites Spektrum von Struktureinheiten mit solchen funktionellen Gruppen enthalten, die sich im Bereich der Optoelektronik und Wirkstofforschung oder zum Aufbau neuer polymerer Materialien nutzen lassen, und die die physikalischen Eigenschaften der Fullerenderivate, wie Löslichkeit oder Polarität, verbessern.

Es ist bekannt, daß aktivierte Olefine, die die Michaelreaktionen eingehen, mit Schwefelyliden (siehe z.B. Trost und Melvin, Sulfur Ylides, Academic Press, New York 1975) und Phosphoryliden (siehe z.B. Bestmann und Seny, Angew. Chem. Int. Ed. Engl. 1 (1962) 116) zu Cyclopropanderivaten reagieren können.

Es wurde nun überraschend gefunden, daß Fullerene mit Phosphoryliden zu methanoüberbrückten Fullerenderivaten reagieren.

Gegenstand der Erfindung ist daher ein Verfahren zur Herstellung eines methanoverbrückten Fullerenderivates, dadurch gekennzeichnet, daß man ein Fulleren oder Fullerenderivat in einem inerten organischen Lösungsmittel bei einer Temperatur von -78 bis 180°C mit einem Phosphorylid umsetzt.

Vorzugsweise werden als Ausgangsverbindungen Fullerene der allgemeinen Formel $C_{20+2m}$, mit m = 2....100, besonders bevorzugt mit m = 20, 25, 28, 29, ganz besonders bevorzugt mit m = 20, 25, eingesetzt.

Als Fullerene werden insbesondere reines $C_{60}$ oder $C_{70}$ eingesetzt.

Es können aber auch Rohfullerene, die ein Gemisch aus $C_{60}$ und $C_{70}$ als Hauptkomponenten enthalten, eingesetzt werden.

Solche Fullerene können beispielsweise durch Herstellung von Fullerenruß im Lichtbogenverfahren mit anschließender Extraktion mit einem unpolaren organischen Lösungsmittel (Rohfulleren), wie z.B. in WO 92/04279 beschrieben, gewonnen werden. Die weitere Feinauftrennung kann säulenchromatographisch erfolgen.

Diese Fullerene sind zum Teil auch Handelsprodukte.

Die eingesetzten Phosphorylide sind im Prinzip bekannt. Sie sind in breitem Rahmen strukturell variierbar. Mögliche Strukturen und deren Synthesen finden sich z.B. in

- Topics in Current Chemistry 20 (1971) 1;
- G. M. Kosolapoff und L. Maier, Organic Phosphorus Compounds, Vol. 3, Kap. 5, Wiley Interscience, New York 1972;
- Chemikerzeitung 96 (1972) 649;
- Carbon-Carbon-Bond Formation, Vol. 1, S. 353 - 426, Verlag Marcel Deccer, New York 1979;
- Angew. Chem. 77 (1985) 651, 850;
- Angew. Chem. 89 (1977) 361;
- Synthesis (1992) 787;
- Houben-Weyl, Methoden der Organischen Chemie E1, Organische Phosphorverbindungen I, S. 616 - 782, Georg Thieme Verlag, Stuttgart 1963;
- B. M. Trost (Ed.), Comprehensive Organic Synthesis, Vol. 6, S. 171 - 202, Pergamon Press, Oxford 1991.

Bevorzugt eingesetzte Ylide sind solche der Formel (I),

$$R^3_3\overset{\oplus}{P}-\overset{\ominus}{C}R^1R^2 \qquad (I)$$

wobei

$R^1$, $R^2$: unabhängig voneinander H, eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH=CH-, -O-, -S-, -COO-, -OOC-, -$SiR^3_2$-, -CO-, Phenylendiyl oder Cyclohexylendiyl ersetzt sein können und wobei ein oder mehrere Wasserstoffatome durch F, Cl, Br, I oder eine Tetrahydropyranyloxygruppe ersetzt sein können, oder $R^1$ und $R^2$ zusammen =C=X, mit X: O, S, $NR^3$, $(O\text{-}R^3)_2$;

$R^3$: gleich oder verschieden Phenyl, $C_1$-$C_{12}$-Alkyl;

bedeuten.

Besonders bevorzugt sind Phosphorylide der allgemeinen Formel (I), bei denen die Symbole folgende Bedeutungen haben:

$R^1$: H, Phenyl;

$R^2$: H, eine geradkettige oder verzweigte (mit oder ohne asymmetrisches Kohlenstoffatom) $C_2$-$C_{20}$-Alkylgruppe, wobei eine oder mehrere $CH_2$-Gruppe durch -C≡C-, -CH=CH-, -O-, -S-, -COO-, -OOC-, -$SiR^3_2$-, -CO-, Phenylendiyl oder Cyclohexylendiyl ersetzt sein können und wobei ein oder mehrere Wasserstoffatome durch F, Cl, Br, I oder eine Tetrahydropyranyloxygruppe ersetzt sein können, oder $R^1$ und $R^2$ zusammen =C=X, mit X: O, S, $NR^3$, $(O\text{-}R^3)_2$;

$R^3$: Phenyl, $C_1$-$C_{12}$-Alkyl.

Ganz besonders bevorzugt sind Phosphorylide der allgemeinen Formel (I), bei denen die Symbole folgende Bedeutungen haben:

$R^1$: H, Phenyl;

$R^2$: H, eine geradkettige oder verzweigte $C_2$-$C_{20}$-Alkylgruppe, wobei eine oder mehrere $CH_2$-Gruppe durch -C≡C-, -CH=CH-, -O-, -S-, -COO-, -OOC-, Phenylendiyl oder Cyclohexylendiyl ersetzt sein können und wobei ein Wasserstoffatom durch eine Tetrahydropyranyloxygruppe ersetzt sein kann;

$R^3$: Phenyl.

Das stöchiometrische Verhältnis von Ylid zu Fulleren beträgt vorzugsweise 8:1 bis 0,5:1, besonders bevorzugt 4:1 bis 0,8:1, ganz besonders bevorzugt 1,5:1 bis 0,9:1.

Zur Herstellung des Monoaddukts von Fulleren und Ylid wird bei annähernder Stöchiometrie der Ausgangsverbindungen vorzugsweise in einem Temperaturbereich von der Raumtemperatur bis zur Siedetemperatur des verwendeten Lösungsmittels bei Normaldruck gearbeitet.

Ein hoher Substitutionsgrad und damit ein großer Wert für n wird erreicht, indem das Ylid im Überschuß eingesetzt wird.

Die Reaktion wird in einem inerten Lösungsmittel durchgeführt. Bevorzugt sind aprotische Lösungsmittel, insbesondere aromatische Kohlenwasserstoffe, wie Benzol, Toluol, Xylole, halogenierte aromatische Kohlenwasserstoffe, wie Chlorbenzol und chlorierte Kohlenwasserstoffe, wie Methylenchlorid. Besonders bevorzugt sind aromatische Kohlenwasserstoffe, insbesondere Benzol und Toluol.

Das Verfahren wird bei einer Temperatur von -78 bis 180°C, bevorzugt 0 bis 150°C, besonders bevorzugt 20 bis 150°C durchgeführt.

In geeigneten Fällen wird bei Raumtemperatur (20 bis 30°C) umgesetzt. Teilweise anfallendes unlösliches zwitterionisches Primäraddukt aus dem Ylid und Fulleren kann beispielsweise durch Erhitzen des Primäraddukts im Hochvakuum bei 250 bis 350°C in das Cyclopropanderivat umgewandelt werden.

In einer bevorzugten Variante zur Durchführung des erfindungsgemäßen Verfahrens wird das Fulleren in dem inerten Lösungsmittel, vorzugsweise ein aromatischer Kohlenwasserstoff, vorgelegt und die entsprechende Menge Phosphoran, vorzugsweise gelöst in dem entsprechenden Lösungsmittel, zugegeben, vorzugsweise langsam unter Rühren.

Das Phosphoran kann vorteilhaft als Maßlösung, hergestellt durch Lösen einer abgewogenen Menge Phosphoran in einer bestimmten Menge des entsprechenden Lösungsmittels, eingesetzt werden.

Die Aufarbeitung erfolgt nach bekannten, dem Fachmann geläufigen Methoden.

Beispielsweise werden etwaige bei der Reaktion entstandene Niederschläge abfiltriert und das Filtrat anschließend eingeengt. Der verbleibende Rückstand kann dann z.B. durch chromatographische Methoden oder Umkristallisation weiter aufgereinigt werden.

Die Produkte des erfindungsgemäßen Verfahrens sind teilweise bekannt und teilweise neu.

Gegenstand der Erfindung sind daher auch Fullerenderivate der Formel (II),

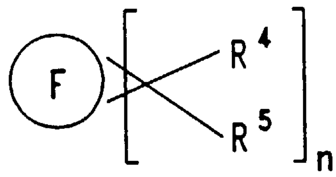

(II)

wobei die Symbole und Indizes folgende Bedeutungen haben:

F: ein Fullerenrest der Formel
$C_{20+2m}$, mit m = 2 bis 100;
$R^4$: H, $CH_3$, -Phenyl, $R^5$;
$R^5$: eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH=CH-, -O-, -S-, -COO-, -OOC-, -$SiR^3_2$-, -CO-, Phenylendiyl oder Cyclohexylendiyl ersetzt sein können und wobei ein oder mehrere Wasserstoffatome durch F, Cl, Br, I, OH oder eine Tetrahydropyranyloxygruppe ersetzt sein können, oder $R^4$ und $R^5$ zusammen =C=X mit X: O, S, $NR^3$, $(O\text{-}R^3)_2$;
$R^3$: Phenyl, $C_1$-$C_{12}$-Alkyl;
n: 1 bis 6.

Bevorzugt sind

F: $C_{60}$, $C_{70}$;
$R^4$: H, Phenyl;
$R^5$: eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH=CH-, -O-, -S-, -COO-, -OOC-, -$SiR^3_2$-, -CO-, Phenylendiyl oder Cyclohexylendiyl ersetzt sein können und wobei ein oder mehrere Wasserstoffatome durch F, Cl, Br, I, OH oder eine Tetrahydropyranyloxygruppe ersetzt sein können, oder $R^4$ und $R^5$ zusammen =C=X mit X: O, S, $NR^3$, $(O\text{-}R^3)_2$;
$R^3$: Phenyl, $C_1$-$C_{12}$-Alkyl;
n: 1.

Besonders bevorzugt sind

F: $C_{60}$, $C_{70}$;
$R^4$: H, Phenyl;
$R^5$: eine geradkettige oder verzweigte $C_2$-$C_{20}$-Alkylgruppe, wobei eine oder mehrere $CH_2$-Gruppen durch -C≡C-, -CH=CH-, -O-, -S-, -OOC-, -COO-, Phenylendiyl oder Cyclohexylendiyl ersetzt sein können und wobei ein Wasserstoffatom durch OH oder eine Tetrahydropyranyloxygruppe ersetzt sein kann;
n: 1.

Erfindungsgemäß hergestellte Fullerenderivate und erfindungsgemäße Fullerenderivate der Formel (II) finden beispielsweise Anwendung in optoelektrischen Bauelementen.

Die Erfindung wird durch die Beispiele näher erläutert.

Beispiele:

Allgemeine Arbeitsvorschrift (AAV)

Alle Umsetzungen von $C_{60}$ mit den Triphenylphosphoranen werden in Benzol oder Toluol durchgeführt. Die Konzentration der $C_{60}$-Lösungen betragen 1 mg/ml. Bei allen Reaktionen bilden die $C_{60}$-Lösungen die Vorlage und die äquimolare Menge (bzw. 10 bis 30 % Überschuß) des jeweiligen Triphenylphosphorans wird als Maßlösung langsam unter Rühren zugetropft. Zur Herstellung der Maßlösungen werden exakt abgewogene Mengen der Triphenylphosphorane in einer bestimmten Menge des entsprechenden Lösungsmittels (Benzol, Toluol) gelöst.

Die bei den Reaktionen entstehenden Niederschläge werden abfiltriert und von den Filtraten die Lösungsmittel abgezogen.

Beispiel 1

Methanofulleren (Umsetzung von $C_{60}$ mit Methylentriphenylphosphoran ($CH_2$=$PPh_3$))

Die Umsetzung erfolgt nach der AAV.
71 mg (0,0986 mmol) $C_{60}$ werden in 70 ml Toluol gelöst und unter Rückfluß erhitzt. Nach Zugabe von 29,2 mg (0,106 mmol) Methylentriphenylphosphoran, gelöst in 3,5 ml Toluol, bildet sich sofort ein Niederschlag. Die Reaktionsmischung wird noch weitere 4 Stunden unter Rückfluß erhitzt und anschließend filtriert.

Aufarbeiten des Filtrates:

Der Rückstand des Filtrates (40 mg) wird nach Abziehen des Lösungsmittels auf eine Kieselgelsäule (Kieselgel 60, Fa. E. Merck, Darmstadt, Deutschland) aufgetragen. Anschließend wird die Säule mit $CS_2$ eluiert (Abtrennung von $PPh_3$). Von der erhaltenen Fraktion ($R_f$: 1) bleibt nach Abzug des Lösungsmittels ein dunkelbrauner mikrokristalliner Rückstand zurück.
Ausbeute: 30 mg
Filterrückstand: 60 mg

Das Massenspektrum des Feststoffes, wie auch des Rückstands des eingedampften Filtrates, zeigt ein Signal bei m/e = 734 = $C_{61}H_2$ und ein weiteres kleineres bei m/e = 748 = $C_{62}H_4$.

Erhitzt man den festen Rückstand im Hochvakuum in einer Sublimationsapparatur auf 250 bis 350°C, so sublimiert Triphenylphosphan ab. Der Rückstand zeigt im Massenspektrum die obengenannten Signale.

Beispiel 2

Methylmethanofulleren (Umsetzung von Ethylidentriphenylphosphoran ($CH_3$-CH=$PPh_3$) mit $C_{60}$):

28 mg (0,039 mmol) $C_{60}$ werden in 25 ml absolutem Benzol oder Toluol gelöst und unter Rückfluß erhitzt. Dazu tropft man eine Lösung von 11,3 mg (0,039 mmol) Ethylidentriphenylphosphoran in 5 ml absolutem Benzol (Maßlösung). Es ist sofort eine Niederschlagbildung zu beobachten. Man kocht noch weitere 6 Stunden und filtriert anschließend. Der Rückstand beträgt 12 mg. Das Filtrat wird eingedampft.

Alle Fraktionen, der Filterrückstand sowie das eingedampfte Filtrat, zeigen im Massenspektrum ein Signal bei m/e = 748 = $C_{60}CHCH_3$ sowie ein wesentlich kleineres bei m/e = 776 = $C_{60}(CHCH_3)_2$.

Erhitzt man den Filterrückstand im Hochvakuum in einer Sublimationsapparatur auf 250 bis 350°C, so sublimiert Triphenylphosphan ab. Im Rückstand kann man im Massenspektrum wiederum die beiden oben genannten Signale sehen.

Beispiel 3

Heptylmethanofulleren (Umsetzung von Octyliden-triphenylphosphoran ($CH_3(CH_2)_6CH$=$PPh_3$) mit $C_{60}$):

Die Umsetzung erfolgt nach der AAV.
100 mg (0,1388 mmol) $C_{60}$ werden in 110 ml Toluol gelöst und zum Rückfluß erhitzt. Nach Zugabe von 57,14 mg (0,1528 mmol) Octylidentriphenylphosphoran, gelöst in 8 ml Toluol (Maßlösung), ist eine geringfügige Trübung der Lösung zu beobachten. Die Reaktionsmischung wird noch 24 Stunden unter Rühren erhitzt und anschließend filtriert.

Aufarbeiten des Filtrats:

Der Rückstand des Filtrats (150 mg) wird nach dem Abziehen des Lösungsmittels auf eine Kieselgelsäure (Kieselgel 60, Fa. E. Merck, Darmstadt, Deutschland) aufgetragen und mit Hexan eluiert. Die gewonnene rotbraune Fraktion ($R_f$: 1) zeigt nach dem Abziehen des Lösungsmittels im Massenspektrum ein Signal bei m/e = 832 = $C_{60}CH(CH_2)_6CH_3$ sowie ein wesentlich kleineres bei m/e = 944 = $C_{60}[CH(CH_2)_6CH_3]_2$.
Ausbeute: 20,8 mg

$^1$H-NMR (400 MHz, $C_6D_6/CS_2$, 27°C): $\underline{\delta}$ = 3,73 (t, $^3\underline{J}$ (H, H) = 7,0 Hz, 1H, CH), 2,25 (q, $^3\underline{J}$ (H, H) = 7,0 Hz, 2H, $CH_2$), 1,79 (m, 2H, $CH_2$), 1,52 (m, 2H, $CH_2$), 1,28 (m, 6H, $CH_2$), 0,90 (mc, 3H, $CH_3$);
$^{13}$C-NMR (100,4 MHz, $C_6D_6/CS_2$ 1:1, 29°C): $\underline{\delta}$ = 150,60, 148,15, 146,15, 145,65, 145,50, 145,44, 145,33, 145,06, 144,53, 144,45, 143,95, 143,04, 142,74, 141,37, 138,33, 136,27 (Fullerensignale), 77,20 (s, 2C, Brückenkopf-C-Atome), 39,82 (s, 1C, CH Brücken-C-Atom), 32,46 (s, 1C, $CH_2$), 30,38 (s, 1C, $CH_2$), 29,85 (s, 1C, $CH_2$), 29,15 (s, 1C, $CH_2$), 26,92 (s, 1C, $CH_2$), 23,41 (s, 1C, $CH_2$), 14,72 (s, 1C, $CH_3$);

EI-MS: 832 ($M^+$), 720 ($[M-CH(CH_2)_6CH_3]^+$).
Filterrückstand: 7 mg

Beispiel 4

Undecylmethanofulleren (Umsetzung von Dodecyliden-triphenylphosphoran ($CH_3(CH_2)_{10}$-CH =$PPh_3$) mit $C_{60}$)

Die Umsetzung erfolgt nach der AAV.
45 mg (0,0625 mmol) $C_{60}$ werden in 45 ml Toluol gelöst und unter Rückfluß erhitzt. Nach Zugabe von 30 mg (0,0697 mmol) Dodecylidentriphenylphosphoran, gelöst in 6 ml Toluol, ist sofort eine Trübung der Lösung zu beobachten. Die Reaktionsmischung wird noch weitere 24 Stunden unter Rückfluß erhitzt und anschließend filtriert.

Aufarbeiten des Filtrates:

Der Rückstand des Filtrates (70 mg) wird nach dem Abziehen des Lösungsmittels auf eine Kieselgelsäure (Kieselgel 60, Fa. E. Merck, Darmstadt, Deutschland) aufgetragen. Mit Hexan als Laufmittel erfolgt die Abtrennung von 12 mg unumgesetztem $C_{60}$. Anschließend wird die Säule mit Benzol eluiert. Die gewonnene rotbraune Fraktion ($R_f$: 1) enthält noch $PPh_3$. Durch eine erneute säulenchromatographische Trennung dieser Fraktion über Kieselgel mit $CS_2$ ($R_f$: 1) als Laufmittel und nach Abziehen des Lösungsmittels bleibt eine braune mikrokristalline Substanz zurück.
Ausbeute: 12 mg
$^1$H-NMR (400 MHz, $C_6D_6/CS_2$, 29°C): $\underline{\delta}$ = 3,68 (t, $^3\underline{J}$ (H, H) = 7,14 Hz, 1H, CH), 2,27 (m, 2H, $CH_2$), 1,77 (m, 2H, $CH_2$),1,50 (m,2H, $CH_2$), 1,29 (m, 14H, $CH_2$), 0,89 (t, $^3\underline{J}$(H, H) = 7,15 Hz, 3H, $CH_3$);
$^{13}$C-NMR (100,4 MHz, $C_6D_6/CS_2$ 1:1, 30°C): $\underline{\delta}$ = 150,60, 148,16, 146,15, 145,65, 145,50, 145,44, 145,33, 145,06, 144,58, 144,50, 144,01, 143,44, 143,04, 142,74, 142,47, 141,37, 138,34, 136,27 (Fullerensignale), 77,35 (s, 2C, Brükkenkopf-C-Atome), 39,73 (s, 1C, CH Brücken-C-Atom), 32,46 (s, 1C, $CH_2$), 30,29 (s, 2C, $CH_2$), 30,26 (s, 2C, $CH_2$), 30,23 (s, 1C,$CH_2$), 29,97 (s, 1C, $CH_2$), 29,90 (s, 1C, $CH_2$), 29,28 (s, 1C, $CH_2$), 23,31 (s, 1C, $CH_2$), 14,55 (s, 1C, $CH_2$),
EI-MS: 888 ($M^+$), 720 ($[M-CH(CH_2)_{10}CH_3]^+$).

Beispiel 5

Decenylmethanofulleren (Umsetzung von Undec-10-en-1-yliden-triphenylphosphoran ($CH_2$=CH-$(CH_2)_8$-CH=$PPh_3$) mit $C_{60}$)

Die Umsetzung erfolgt nach der AAV.
62 mg (0,086 mmol) $C_{60}$ werden in 62 ml Toluol gelöst und unter Rückfluß erhitzt. Nach Zugabe von 43,5 mg (0,105 mmol) Undecenylidentriphenylphosphoran, gelöst in 7 ml Toluol, ist sofort eine Trübung der Lösung zu beobachten. Die Reaktionsmischung wird noch weitere 24 Stunden unter Rückfluß erhitzt und anschließend filtriert.

Aufarbeiten des Filtrates:

Der Rückstand des Filtrates (92 mg) wird nach Abziehen des Lösungsmittels auf eine Kieselgelsäule (Kieselgel 60, Fa. E. Merck, Darmstadt, Deutschland) aufgetragen. Anschließend wird die Säule mit $CS_2$ eluiert (Abtrennung von $PPh_3$). Von der erhaltenen Fraktion ($R_f$: 0,95) bleibt nach Abzug des Lösungsmittels ein dunkelbrauner mikrokristalliner Rückstand zurück.
Ausbeute: 34 mg
$^1$H-NMR (400 MHz, $CS_2/CSCl_3$, 29°C): $\underline{\delta}$ = 5,78 (ddt, $^3\underline{J}$(H, H) = 17,9 Hz, $^3\underline{J}$(H, H) = 10,3 Hz, $^3\underline{J}$(H, H) = 6,72 Hz, 1H, CH), 4,99 (dd, $^3\underline{J}$(H, H) = 17,9 Hz, $^2\underline{J}$(H, H) = 1,83 Hz, 1H, CH), 4,92 (dd, $^3\underline{J}$(H, H) = 10,3 Hz, $^2\underline{J}$(H, H) = 1,83 Hz, 1H, CH), 4,07 (t, $^3\underline{J}$(H, H) = 7,63 Hz, 1H, CH), 2,54 (dt, $^3\underline{J}$(H, H) = 7,63 Hz, $^3\underline{J}$(H, H) = 7,63 Hz, 2H, $CH_2$), 2,06 bis 1,96 (m, 6H, $CH_2$), 1,8 bis 1,2 (m, 8H, $CH_2$);
$^{13}$C-NMR (100,4 MHz, $CS_2/CDCl_3$ 2:1, 30°C): $\underline{\delta}$ = 150,09, 147,63, 145,68, 145,17, 144,97, 144,90, 144,79, 144,53, 144,46, 144,05, 143,95, 143,47, 142,79, 142,51, 142,19, 141,95, 141,92, 140,84, 140,80, 137,84, 135,77 (Fullerensignale), 138,67 (s, 1C, $CH_2$), 114,23 (s, 1C, =$CH_2$), 76,80 (s, 2C, Brückenkopf-C-Atome), 39,42 (s, 1C, CH Brücken-C-Atom), 33,89 (s, 1C, $CH_2$), 29,78 (s, 1C, $CH_2$), 29,70 (s, 1C, $CH_2$), 29,64 (s, 2C, $CH_2$), 29,58 (s, 1C, $CH_2$), 29,28 (s, 1C, $CH_2$), 29,05 (s, 1C, $CH_2$);
EI-MS: 872 ($M^+$), 720 ($[M-CH(CH_2)_8-CH=CH_2]^+$)
Filterrückstand: 3 mg

Beispiel 6

Phenylmethanofulleren (Umsetzung von Benzyliden-triphenylphosphoran ($C_6H_5$-CH=$PPh_3$) mit $C_{60}$)

Die Umsetzung erfolgt nach der AAV.
30 mg (0,0416 mmol) $C_{60}$ werden in 30 ml Benzol gelöst. Nach Zugabe von 15 mg (0,0426 mmol) Benzyliden-triphenylphosphoran, gelöst in 1 ml Benzol, ist eine Trübung der Lösung zu beobachten. Die Reaktionsmischung wird noch weitere 24 Stunden bei Raumtemperatur gerührt und anschließend filtriert.

Aufarbeiten des Filtrates:

Der Rückstand des Filtrates (41 mg) wird nach Abziehen des Lösungsmittels auf eine Kieselgelsäule (Kieselgel 60, Fa. E. Merck, Darmstadt, Deutschland) aufgetragen. Mit Hexan als Laufmittel erfolgt die Abtrennung von unumgesetztem $C_{60}$. Anschließend wird die Säule mit Benzol eluiert. Die gewonnene rotbraune Fraktion ($R_f$: 1) enthält noch $PPh_3$. Durch eine erneute säulenchromatographische Trennung dieser Fraktion über Kieselgel mit Benzol ($R_f$: 1) als Laufmittel und nach Abziehen des Lösungsmittels bleibt nun eine braune mikrokristalline Substanz zurück.
Ausbeute: 10 mg
$^1$H-NMR (400 MHz, $CHCl_3/CS_2$ 1:1, 27°C): $\underline{\delta}$ = 7,92 (d, $^3\underline{J}$(H, H) = 7,02 Hz, 2H, o-H(Ph)), 7,49 (t, $^3\underline{J}$(H, H) = 7,32 Hz, 2H, m-H(Ph)), 7,41 (t, $^3\underline{J}$(H, H) = 7,02 Hz, 1H, p-H(Ph)), 5,36 (s, 1H, CH);
$^{13}$C-NMR (100,4 MHz, $CDCl_3/CS_2$ 1:1, 27°C): $\underline{\delta}$ = 133 bis 150 (Fullerensignale), 140,93 (s, 1C, C(Ph), 131,02 (s, 2C, C(Ph)), 128,62 (s, 2C, C(Ph)), 128,26 (s, 1C, C(Ph)), 75,30 (s, 2C, Brückenkopf-C-Atome), 43,37 (s, 1C, CH Brücken-C-Atom);
EI-MS: 810 ($M^+$), 720 ($[M\text{-}CHPh]^+$)
Filterrückstand: 4 mg

Beispiel 7

Diphenylmethanofulleren (Umsetzung von Diphenylmethylentriphenylphosphoran ($(C_6H_5)_2C$=$PPh_3$) mit $C_{60}$)

Die Umsetzung erfolgt nach der AAV.
28 mg (0,039 mmol) $C_{60}$ werden in 28 ml Toluol gelöst und unter Rückfluß erhitzt. Nach Zugabe von 16,6 mg (0,039 mmol) Diphenylmethylentriphenylphosphoran, gelöst in 8,2 mmol Benzol, ist eine Trübung der Lösung zu beobachten. Die Reaktionsmischung wird noch weitere 24 Stunden unter Rückfluß erhitzt und anschließend filtriert.

Aufarbeiten des Filtrates:

Der Rückstand des Filtrates (39 mg) wird nach Abziehen des Lösungsmittels auf eine Kieselgelsäule (Kieselgel 60, Fa. E. Merck, Darmstadt, Deutschland) aufgetragen. Mit Hexan als Laufmittel erfolgt die Abtrennung von 5 mg unumgegesetztem $C_{60}$. Anschließend wird die Säule mit Benzol eluiert. Die gewonnene rotbraune Fraktion ($R_f$: 1) enthältnoch $PPh_3$. Durch eine erneute säurenchromatographische Trennung dieser Fraktion über Kieselgel mit Benzol ($R_f$: 1) als Laufmittel und nach Abziehen des Lösungsmittels bleibt nun eine braune mikrokristalline Substanz zurück.
$^1$H-NMR (400 MHz, $CHCl_3/CS_2$ 1:1, 27°C): $\underline{\delta}$ = 7,97 (d, $^3\underline{J}$(H, H) = 7,02 Hz, o-H(Ph)), 7,36 (t, $^3\underline{J}$(H, H) = 7,32 Hz, 4H, m-H(Ph)), 7,26 (t, $^3\underline{J}$(H, H) = 7,32 Hz, 2H, p-H(Ph));
EI-MS: 886 ($M^+$), 720 ($[M\text{-}CHPh_2]^+$).
Filterrückstand: 5,6 mg

Beispiel 8

Propansäureethylestermethanofulleren (Umsetzung von 3-Carboethoxypropyliden-triphenylphosphoran ($H_5C_2$-O-CO-$CH_2$-$CH_2$-CH=$PPh_3$) mit $C_{60}$)

Die Umsetzung erfolgt nach der AAV.
65 mg (0,0903 mmol) $C_{60}$ werden in 65 ml Toluol gelöst und unter Rückfluß erhitzt. Nach Zugabe von 35 mg (0,0931 mmol) 3-Carboethoxypropylidentriphenylphosphoran, gelöst in 10 ml Toluol, ist sofort eine Niederschlagsbildung zu beobachten. Die Reaktionsmischung wird noch weitere 24 Stunden unter Rückfluß erhitzt und anschließend filtriert.

Aufarbeiten des Filtrates:

Der Rückstand des Filtrates (59 mg) wird nach Abziehen des Lösungsmittels auf eine Kieselgelsäule (Kieselgel 60, Fa. E. Merck, Darmstadt, Deutschland) aufgetragen. Mit $CS_2$ als Laufmittel erfolgt die Abtrennung von unumgesetztem $C_{60}$ ($R_f$: 1) und entstandenem $PPh_3$ ($R_f$: 0,4). Anschließend wird die Säule mit $CH_2Cl_2$ eluiert. Von der erhaltenen Fraktion ($R_f$: 0,9) bleibt nach Abziehen des Lösungsmittels ein dunkelbrauner mikrokristalliner Rückstand zurück.
Ausbeute: 15 mg

$^1$H-NMR (400 MHz, $CDCl_3$, 28°C): $\underline{\delta}$ = 4,27 (q, $^3\underline{J}$(H, H) = 7,33 Hz, 2H, $OC\underline{H}_2CH_3$), 4,20 (t, $^3\underline{J}$(H, H) = 7,36 Hz, 1H, $C\underline{H}$-$CH_2$), 2,97 (t, $^3\underline{J}$(H, H) = 7,94 Hz, 2H, $C\underline{H}_2$COOEt), 2,86 (dt, $^3\underline{J}$(H, H) = 7,63 Hr, $^3\underline{J}$(H, H) = 6,56 Hz, 2H, CH-$C\underline{H}_2$-$CH_2$. 1,34 (t, $^3\underline{J}$(H, H) = 7,02 Hz, 3H, $CH_3$);
$^{13}$C-NMR (100,4 MHz, $CDCl_3$, 29°C): $\underline{\delta}$ = 172,63 (s, 1C, $\underline{C}$OOEt), 150,03, 147,31, 145,85, 145,28, 145,20, 145,14, 145,02, 144,82, 144,76, 144,67, 144,32, 144,27, 144,18, 143,68, 143,11, 142,98, 142,67, 142,38, 142,15, 142,12, 141,00, 138,10, 136,09 (Fullerensignale), 76,33 (s, 2C, Brückenkopf-C-Atome), 60,99 (s, 1C, O-$\underline{C}H_2CH_3$), 37,94 (s, 1C, $\underline{C}$H Brücken-C-Atom), 33,24 (s, 1C, $CH_2\underline{C}H_2$-COOEt), 22,08 (s, 1C, $\underline{C}H_2$-COOEt), 14,33 (s, 1C, $CH_3$);
EI-MS: 834 ($M^+$), 720 ($[M\text{-}CH(CH_2)_2COOEt]^+$).
Filterrückstand: 41 mg

Beispiel 9

Undecansäuremethylestermethanofulleren (Umsetzung von Carboethoxyundecyliden-triphenylphosphoran ($H_5C_2OOC$-$(CH_2)_{10}$-$CH{=}PPh_3$) mit $C_{60}$)

Die Umsetzung erfolgt nach der AAV.
65 mg (0,0903 mmol) $C_{60}$ werden in 65 ml Toluol gelöst und unter Rückfluß erhitzt. Nach Zugabe von 35 mg (0,0931 mmol) 3-Carboethoxyundecylidentriphenylphosphoran, gelöst in 10 ml Toluol, ist sofort eine Niederschlagsbildung zu beobachten. Die Reaktionsmischung wird noch weitere 24 Stunden unter Rückfluß erhitzt und anschließend filtriert.

Aufarbeiten des Filtrates:

Der Rückstand des Filtrates (59 mg) wird nach Abziehen des Lösungsmittels auf eine Kieselgelsäule (Kieselgel 60, Fa. E. Merck, Darmstadt) aufgetragen. Mit $CS_2$ als Laufmittel erfolgt die Abtrennung von unumgesetztem $C_{60}$ ($R_f$: 1) und entstandenem $PPh_3$ ($R_f$: 0,4). Anschließend wird die Säule mit $CH_2Cl_2$ eluiert. Von der erhaltenen Fraktion ($R_f$: 0,9) bleibt nach Abziehen des Lösungsmittels ein dunkelbrauner mikrokristalliner Rückstand zurück.
Ausbeute: 15 mg

$^1$H-NMR (400 MHz, $CDCl_3$, 28°C): $\underline{\delta}$ = 4,27 (q, $^3\underline{J}$(H, H) = 7,33 Hz, 2H, $OC\underline{H}_2CH_3$), 4,20 (t, $^3\underline{J}$(H, H) = 7,36 Hz, 1H, $C\underline{H}$-$CH_2$), 2,97 (t, $^3\underline{J}$(H, H) = 7,94 Hz, 2H, $C\underline{H}_2$COOEt), 2,86 (dt, $^3\underline{J}$(H, H) = 7,63 Hr, $^3\underline{J}$(H, H) = 6,56 Hz, 2H, CH-$C\underline{H}_2$-$CH_2$. 1,34 (t, $^3\underline{J}$(H, H) = 7,02 Hz, 3H, $CH_3$);
$^{13}$C-NMR (100,4 MHz, $CDCl_3$, 29°C): $\underline{\delta}$ = 172,63 (s, 1C, $\underline{C}$OOEt), 150,03, 147,31, 145,85, 145,28, 145,20, 145,14, 145,02, 144,82, 144,76, 144,67, 144,32, 144,27, 144,18, 143,68, 143,11, 142,98, 142,67, 142,38, 142,15, 142,12, 141,00, 138,10, 136,09 (Fullerensignale), 76,33 (s, 2C, Brückenkopf-C-Atome), 60,99 (s, 1C, O-$\underline{C}H_2CH_3$), 37,94 (s, 1C, $\underline{C}$H Brücken-C-Atom), 33,24 (s, 1C, $CH_2$-$\underline{C}H_2$-COOEt), 22,08 (s, 1C, $\underline{C}H_2$-COOEt), 14,33 (s, 1C, $CH_3$);
EI-MS: 834 ($M^+$), 720 ($[M\text{-}CH(CH_2)_2COOEt]^+$).
Filterrückstand: 41 mg

Beispiel 10

Undecansäuremethylestermethanofulleren (Umsetzung von Carboethoxyundecyliden-triphenylphosphoran ($H_5C_2OOC$-$(CH_2)_{10}$-$CH{=}PPh_3$) mit $C_{60}$)

Die Umsetzung erfolgt nach der AAV.
90 mg (0,125 mmol) $C_{60}$ werden in 100 ml Toluol gelöst und unter Rückfluß erhitzt. Nach Zugabe von 70 mg (0,147 mmol) Carboethoxyundecylidentriphenylphosphoran, gelöst in 25 ml Toluol, ist sofort eine Niederschlagbildung zu beobachten. Die Reaktionsmischung wird noch weitere 24 Stunden unter Rückfluß erhitzt und anschließend filtriert.

Aufarbeiten des Filtrates:

Der Rückstand des Filtrates (50 mg) wird nach Abzug des Lösungsmittels auf eine Kieselgelsäule (Kieselgel 60, Fa. E. Merck, Darmstadt, Deutschland) aufgetragen. Mit $CS_2$ als Laufmittel erfolgt die Abtrennung von unumgesetztem

$C_{60}$ ($R_f$: 1) und entstandenem $PPh_3$ ($R_f$: 0,4). Anschließend wird die Säule mit $CH_2Cl_2$ eluiert. Von der erhaltenen Fraktion ($R_f$: 1) bleibt nach Abzug des Lösungsmittels ein dunkelbrauner mikrokristalliner Rückstand zurück.
Ausbeute: 14 mg

$^1$H-NMR (400 MHz, $CDCl_3$, 30°C): $\underline{\delta}$ = 4,03 (t, $^3\underline{J}$(H, H) = 7,63 Hz, 1H, C-H), 3,60 (s, 3H, O-$CH_3$), 2,49 (dt, $^3\underline{J}$(H, H) = 7,63 Hz, $^3\underline{J}$(H, H) = 7,93 Hz, 2H, CH-C$\underline{H_2}$-$CH_2$), 2,25 (t, $^3\underline{J}$(H, H) = 7,63 Hz, 2H, C$\underline{H_2}$-COOMe), 1,93 (m, 2H, $CH_2$), 1,60 (m, 4H, $CH_2$), 1,27 bis 1,42 (m, 10H, $CH_2$);
$^{13}$C-NMR (100,4 MHz, $CDCl_3$, 30°C): $\underline{\delta}$ = 174,34 (s, 1C, $\underline{C}$OOMe), 150,47, 148,04, 145,94, 145,43, 145,17, 145,11, 145,06, 144,73, 144,54, 144,26, 144,17, 144,11, 143,67, 142,97, 142,73, 142,42, 142,15, 142,10, 140,96, 138,01, 135,94 (Fullerensignale), 77,74 (s, 2C, Brückenkopf-C-Atome), 51,47 (s, 1C, O-$\underline{C}H_3$), 39,46 (s, 1C, $\underline{C}$-H Brücken-C-Atom), 34,12 (s, 1C, $\underline{C}H_2$-COOMe), 29,61 (s, 1C, $CH_2$), 29,57 (s, 1C, $CH_2$), 29,46 (s, 1C, $CH_2$), 29,40 (s, 2C, $CH_2$), 29,28 (s, 1C, $CH_2$), 17,57 (s, 1C, $CH_2$), 28,87 (s, 1C, $CH_2$);
EI-MS: 921 ($M^+$), 720 ($[M\text{-}CH(CH_2)_{10}COOMe]^+$).
Rückstand: 110 mg

Beispiel 11

8-(Tetrahydro-2-pyranyloxy)-octylmethanofulleren und 8-Hydroxyoctylmethanofulleren (Umsetzung von 9-(Tetrahydro-2-pyranyloxy)non-1 yliden-triphenylphosphoran (THP-O-$(CH_2)_8$-CH=$PPh_3$) mit $C_{60}$)

Die Umsetzung erfolgt nach der AAV.
60 mg (0,083 mmol) $C_{60}$ werden in 60 ml Toluol gelöst und unter Rückfluß erhitzt. Nach Zugabe von 41 mg (0,084 mmol) 9-(Tetrahydro-2-pyranyloxy)nonyliden-triphenylphosphoran, gelöst in 2 ml Toluol, wird die Reaktionsmischung noch weitere 24 Stunden unter Rückfluß erhitzt, wobei sich die Lösung langsam eintrübt, und anschließend filtriert.

Aufarbeiten des Filtrates:

Der Rückstand des Filtrates (88 mg) wird nach Abziehen des Lösungsmittels auf eine Kieselgelsäule (Kieselgel 60, Fa. E. Merck, Darmstadt, Deutschland) aufgetragen. Mit $CS_2$ als Laufmittel erfolgt die Abtrennung von 17 mg unumgesetztem $C_{60}$ ($R_f$: 1,0). Anschließend wird die Säule mit Methylenchlorid eluiert. Dabei werden nach Abziehen des Lösungsmittels zwei Fraktionen gewonnen, zuerst 21 mg einer rotbraunen Fraktion mit $R_f$: 0,45 (Fraktion 1), anschließend 24 mg einer hellbraunen Fraktion mit $R_f$: 0,2 (Fraktion 2). Bei einer erneuten säulenchromatographischen Trennung der Fraktion 1 über Kieselgel mit Methylenchlorid als Laufmittel kommt es zur selben Abspaltung in zwei Fraktionen wie bei der ersten Elution mit Methylenchlorid, was auf eine Spaltung des Ethers auf der Säule schließen läßt. Die vereinigten Fraktionen 2 wurden zur Reinigung abschließend dreimal mit je 2 ml Diethylether gewaschen, in 3 ml $CS_2$ gelöst und durch langsames Zutropfen von Diethylether wieder ausgefällt.

Das Massenspektrum der Fraktion 1 zeigt ein Signal bei m/e = 862, welches dem entsprechenden Alkohol des gespaltenen Tetrahydropyranylethers (8-Hydroxyoctylmethanofulleren) entspricht. Im $^1$H-NMR ist aber deutlich das Aufspaltungsmuster des Tetrahydropyranyl-Hydroxyoctylmethanofulleren zu sehen. Auch der in Chloroform lösliche Niederschlag zeigt im Massenspektrum das Signal bei m/e = 862. Der dazugehörige Alkohol ist aber im $^1$H-NMR nicht zu erkennen.

Ausbeute an 8-(Tetrahydro-2-pyranyloxy)-octylmethanofulleren (Fraktion 1): 21 mg

$^1$H-NMR (400 MHz, $CDCl_3$, 29°C): $\underline{\delta}$ = 4,6 (m, 1H, OCHO), 4,11 (t, $^3\underline{J}$(H, H) = 7,47 Hz, 1H, CH), 3,4 bis 3,95 (m, 4H, $CH_2O$), 2,56 (dt, $^3\underline{J}$(H, H) = 7,53 Hz, 2H, $CH_2$), 2,00 (m, 2H, $CH_2$), 1,3 bis 1,9 (m, 16H, $CH_2$);
$^{13}$C-NMR (100,4 MHz, $CDCl_3$, 30°C): $\underline{\delta}$ = 150,46, 148,02, 145,94, 145,43, 145,17, 145,06, 144,97, 144,71, 144,26, 144,17, 144,11, 143,66, 142,95, 142,71 142,42, 142,15, 140,93, 138,00, 135,93 (Fullerensignale), 98,90 (s, 1C, OCHO), 77,62 (s, 2C, Brückenkopf-C-Atome), 67,58 (s, 1C, $CH_2O$), 62,43 (s, 1C, $CH_2O$), 39,72 (s, 1C, CH Brücken-C-Atom), 29,78 (s, 1C, $CH_2$), 29,47 (s, 1C, $CH_2$), 29,35 (s, 1C, $CH_2$), 28,91 (s, 1C, $CH_2$), 28,68 (s, 1C, $CH_2$), 26,41 (s, 1C, $CH_2$), 26,29 (s, 1C, $CH_2$), 25,51 (s, 2C, $CH_2$), 19,76 (s, 1C, $CH_2$);
EI-MS: 862 ($[M\text{-}C_5H_8\text{(Dihydropyran)}]^+$), 720 ($[M\text{-}CH(CH_2)_8\text{-}OTHP]^+$).

Ausbeute an 8-Hydroxyoctylmethanofulleren (Fraktion 2): 24 mg $^1$H-NMR (400 MHz, $CH_2/C_6D_6$ 5:1, 29°C): $\underline{\delta}$ = 4,89 (m, 1H, OH), 3,9 (t, $^3\underline{J}$(H, H) = 7,49 Hz, 1H, CH), 3,41 (t, $^3\underline{J}$(H, H) = 6,43 Hz, 2H, $CH_2O$), 2,41 (dt, $^3\underline{J}$(H, H) = 7,54 Hz, 2H, $CH_2$), 1,88 (m, 2H, $CH_2$), 1,57 (m, 2H, $CH_2$), 1,5 bis 1,2 (m, 8H, $CH_2$);
$^{13}$C-NMR (100,4 MHz, $CS_2/C_6D_6$ 5:1, 31°C): $\underline{\delta}$ = 150,43, 147,96, 146,08, 145,56, 145,43, 145,37, 145,24, 144,97, 144,89, 144,50, 144,42, 143,92, 143,24, 142,95, 142,62, 142,36, 141,31, 141,27, 138,26, 136,21 (Fullerensignale), 77,21 (s, 2C, Brückenkopf-C-Atome), 62,85 (s, 1C, $CH_2O$), 39,84 (s, 1C, CH Brücken-C-Atom), 33,48 (s, 1C, $CH_2$), 30,40 (s, 1C, $CH_2$), 30,08 (s, 1C, $CH_2$), 29,53 (s, 2C, $CH_2$), 26,98 (s, 1C, $CH_2$), 26,51 (s, 1C, $CH_2$);
EI-MS: 862 ($M^+$), 720 ($[M\text{-}CH(CH_2)_8\text{-}OH]^+$).
Filterrückstand: 15 mg

Beispiel 12

Phenylthiomethanofulleren (Umsetzung von Phenylthiomethylentriphenylphosphoran ($C_6H_5SCH{=}PPh_3$) mit $C_{60}$

Die Umsetzung erfolgt nach der AAV.
112 mg (0.155 mmol) $C_{60}$ werden in 125 ml Toluol gelöst und unter Rückfluß erhitzt. Nach Zugabe von 59,74 mg (0,155 mmol) Phenylthiomethylentriphenylphosphoran in 10 ml Toluol (Maßlösung) ist keine Trübung zu beobachten; diese tritt in äußerst geringem Maße erst nach 24stündigem Erhitzen unter Rückfluß auf. Die Reaktionsmischung wird anschließend filtriert.

Aufarbeiten des Filtrats:

Der Rückstand des Filtrats (171 mg) wird nach dem Abziehen des Lösungsmittels auf eine Kieselgelsäule (Kieselgel 60, Fa. E. Merck, Darmstadt, Deutschland) aufgetragen. Durch Elution mit $CS_2$ kann das Produkt ($R_f$: 0,85) von nichtumgesetztem $C_{60}$ ($R_f$: 0,92) und Triphenylphosphan abgetrennt werden. Nach dem Abziehen des Lösungsmittels bleibt eine braune mikrokristalline Substanz zurück.
Ausbeute: 31,3 mg
$^1$H-NMR (400 MHz, $C_6D_6/CS_2$, 29°C): $\delta$ = 7,63 (d, $^3J$(H, H) = 7,40 Hz, 2H, o-H(Ph)), 7,19 (t, $^3J$(H, H) = 7,60 Hz, 2H, m-H(Ph)), 7,07 (t, $^3J$(H, H) = 7,40 Hz, 1H, p-H(Ph)), 5,06 (s, 1H, CH-S);
$^{13}$C-NMR (100,4 MHz, $C_6D_6/CS_2$ 1:1, 29°C): $\delta$ = 148,63, 146,72, 145,94, 145,64, 145,59, 145,50, 145,08, 145,02, 144,89, 144,12, 144,09, 143,51, 143,24, 142,85, 142,78, 142,42, 141,40, 141,31, 140,11, 137,03, 135,41 (Fullerensignale), 129,83 (s, 2C, C(Ph)), 129,70 (s, 2C, C(Ph)), 128,56 (s, 1C, C(Ph)), 127,50 (s, 1C, C(Ph)), 74,43 (s, 2C, Brückenkopf-C-Atome), 39,39 (s, 1C, CH-S-Brücken-C-Atom);
EI-MS: 842 ($M^+$), 733 ($[M-SPh]^+$), 720 ($[M-CHSPh]^+$).

## Patentansprüche

1. Verfahren zur Herstellung eines methanoverbrückten Fullerenderivates, dadurch gekennzeichnet, daß man ein Fulleren oder Fullerenderivat in einem inerten organischen Lösungsmittel bei einer Temperatur von -78 bis 180°C mit einem Phosphorylid umsetzt.

2. Verfahren nach Anspruch 1, dadurch gekennzeichnet, daß ein Fulleren der allgemeinen Formel $C_{20+2m}$, mit m = 2....100, eingesetzt wird.

3. Verfahren nach Anspruch 1 und/oder 2, dadurch gekennzeichnet, daß ein Ylid der allgemeinen Formel (I) eingesetzt wird,

$$R^3_3\overset{\oplus}{P}-\overset{\ominus}{C}R^1R^2 \qquad (I)$$

wobei

R[1], R[2]: unabhängig voneinander H, eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH=CH-, -O-, -S-, -OOC-, -COO-, $-SiR^3_2-$, -CO-, Phenylendiyl oder Cyclohexylendiyl ersetzt sein können und wobei ein oder mehrere Wasserstoffatome durch F, Cl, Br, I oder eine Tetrahydropyranyloxygruppe ersetzt sein können, oder $R^1$ und $R^2$ zusammen =C=X, mit X: O, S, $NR^3$, $(O-R^3)_2$;
$R^3$: gleich oder verschieden Phenyl, $C_1$-$C_{12}$-Alkyl;

bedeuten.

4. Verfahren nach einem oder mehreren der Ansprüche 1 bis 3, dadurch gekennzeichnet, daß das stöchiometrische Verhältnis von Ylid zu Fulleren 8:1 bis 0,5:1 beträgt.

**5.** Verfahren nach einem oder mehreren der Ansprüche 1 bis 4, dadurch gekennzeichnet, daß ein inertes Lösungsmittel aus der Gruppe Benzol, Toluol, Chlorbenzol und Methylenchlorid verwendet wird.

**6.** Fullerenderivat der Formel (II),

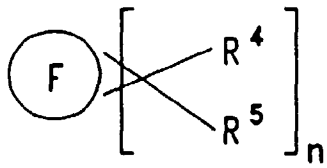

(II)

worin die Symbole und Indizes folgende Bedeutungen haben:

F: ein Fullerenrest der Formel $C_{20+2m}$, mit m = 2 bis 100;
$R^4$: H, $CH_3$, -Phenyl, $R^5$;
$R^5$: eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH=CH-, -O-, -S-, -OOC-, -COO-, -$SiR^3_2$-, -CO-, Phenylendiyl oder Cyclohexylendiyl ersetzt sein können und wobei ein oder mehrere Wasserstoffatome durch F, Cl, Br, I, OH oder eine Tetrahydropyranyloxygruppe ersetzt sein können, oder $R^4$ und $R^5$ zusammen =C=X, mit X: O, S, $NR^3$, $(O\text{-}R^3)_2$;
$R^3$: Phenyl, $C_1$-$C_{12}$-Alkyl;
n: 1 bis 6.

**7.** Fullerenderivat nach Anspruch 6, dadurch gekennzeichnet, daß die Symbole und Indizes in der allgemeinen Formel (II) folgende Bedeutung haben:

F: $C_{60}$, $C_{70}$;
$R^4$: H, Phenyl;
$R^5$: eine geradkettige oder verzweigte (mit oder ohne asymetrisches Kohlenstoffatom) $C_2$-$C_{20}$-Alkylgruppe, wobei eine oder mehrere $CH_2$-Gruppen durch -C≡C-, -CH = CH-, -O-, -S-, -COO-, -OOC-, -$SiR^3_2$-, -CO-, Phenylendiyl oder Cyclohexylendiyl ersetzt sein können und wobei ein oder mehrere Wasserstoffatome durch F, Cl, Br, I, OH oder eine Tetrahydropyranyloxygruppe ersetzt sein können, oder $R^4$ und $R^5$ zusammen =C=X mit X: O, S, $NR^3$, $(O\text{-}R^3)_2$;
$R^3$: Phenyl, $C_1$-$C_{12}$-Alkyl;
n: 1.

**8.** Fullerenderivat nach Anspruch 6 und/oder 7, dadurch gekennzeichnet, daß die Symbole und Indizes in der allgemeinen Formel (II) folgende Bedeutungen haben:

F: $C_{60}$, $C_{70}$;
$R^4$: H, Phenyl;
$R^5$: eine geradkettige oder verzweigte $C_2$-$C_{20}$-Alkylgruppe, wobei eine oder mehrere $CH_2$-Gruppen durch -C≡C-, -CH=CH-, -O-, -S-, -COO-, -OOC-, Phenylendiyl oder Cyclohexylendiyl ersetzt sein können und wobei ein Wasserstoffatom durch OH oder eine Tetrahydropyranyloxygruppe ersetzt sein kann;
n: 1.

**9.** Verwendung von methanoverbrückten Fullerenderivaten, hergestellt mit einem Verfahren nach einem oder mehreren der Ansprüche 1 bis 5, in optoelektrischen Bauelementen.

**10.** Verwendung von Fullerenderivaten der allgemeinen Formel (II) nach einem oder mehreren der Ansprüche 6 bis 8 in optoelektrischen Bauelementen.

## Claims

1. A process for preparing a methano-bridged fullerene derivative, which comprises reacting a fullerene or fullerene derivative with a phosphorus ylide in an inert organic solvent at a temperature of from -78 to 180°C.

2. The process as claimed in claim 1, wherein a fullerene of the formula $C_{20+2m}$, with m = 2....100, is employed.

3. The process as claimed in claim 1 and/or 2, wherein an ylide of the formula (I) is employed,

$$\overset{\oplus}{R^3_3P}-\overset{\ominus}{C}R^1R^2 \qquad (I)$$

where

$R^1$, $R^2$: are, independently of each other, H, a straight-chain or branched (with or without an asymmetric carbon atom) $C_2$-$C_{20}$-alkyl group, where one or more $CH_2$ groups can be replaced by -C≡C-, -CH=CH-, -O-, -S-, -OOC-, -COO-, -Si$R^3_2$-, -CO-, phenylenediyl or cyclohexylenediyl, and where one or more hydrogen atoms can be replaced by F, Cl, Br, I or a tetrahydropyranyloxy group, or $R^1$ and $R^2$ together are =C=X, with X being O, S, $NR^3$ or $(O\text{-}R^3)_2$;
$R^3$: is, identically or differently, phenyl or $C_1$-$C_{12}$-alkyl.

4. The process as claimed in one or more of claims 1 to 3, wherein the stoichiometric ratio of ylide to fullerene is from 8:1 to 0.5:1.

5. The process as claimed in one or more of claims 1 to 4, wherein an inert solvent from the group benzene, toluene, chlorobenzene and methylene chloride is used.

6. A fullerene derivative of the formula (II),

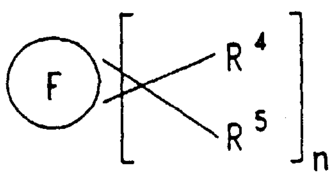

(II)

in which the symbols and indices have the following meanings:

F: is a fullerene radical of the formula $C_{20\,+\,2m}$, with m = 2 to 100;
$R^4$: is H, $CH_3$, -phenyl or $R^5$;
$R^5$: is a straight-chain or branched (with or without an asymmetric carbon atom) $C_2$-$C_{20}$-alkyl group, where one or more $CH_2$ groups can be replaced by -C≡C-, -CH=CH-, -O-, -S-, -OOC-, -COO-, -Si$R^3_2$-, -CO-, phenylenediyl or cyclohexylenediyl, and where one or more hydrogen atoms can be replaced by F, Cl, Br, I, OH or a tetrahydropyranyloxy group, or $R^4$ and $R^5$ together are =C=X, with X being O, S, $NR^3$ or $(O\text{-}R^3)_2$;
R : is phenyl or $C_1$-$C_{12}$-alkyl;
n: is from 1 to 6.

7. A fullerene derivative as claimed in claim 6, wherein the symbols and indices in the formula (II) have the following meaning:

F: is $C_{60}$ or $C_{70}$;
$R^4$: is H or phenyl;
$R^5$: is a straight-chain or branched (with or without an asymmetric carbon atom) $C_2$-$C_{20}$-alkyl group, where one or more $CH_2$ groups can be replaced by -C≡C-, -CH=CH-, -O-, -S-, -COO-, -OOC-, -Si$R^3_2$-, -CO-, phenylenediyl or cyclohexylenediyl, and where one or more hydrogen atoms can be replaced by F, Cl, Br, I, OH or a tetrahydropyranyloxy group, or $R^4$ and $R^5$ together are =C=X with X being O, S, $NR^3$ or $(O\text{-}R^3)_2$;

$R^3$: is phenyl or $C_1$-$C_{12}$-alkyl;
n: is 1.

**8.** A fullerene derivative as claimed in claim 6 and/or 7, wherein the symbols and indices in the formula (II) have the following meanings:

F: is $C_{60}$ or $C_{70}$;
$R^4$: is H or phenyl;
$R^5$: is a straight-chain or branched $C_2$-$C_{20}$-alkyl group, where one or more $CH_2$ groups can be replaced by -C≡C-, -CH=CH-, -O-, -S-, -COO-, -OOC-, phenylenediyl or cyclohexylenediyl, and where one hydrogen atom can be replaced by OH or a tetrahydropyranyloxy group;
n: is 1.

**9.** Use of methano-bridged fullerene derivatives, prepared using a process as claimed in one or more of claims 1 to 5, in optoelectrical units.

**10.** Use of fullerene derivatives of the formula (II) as claimed in one or more of claims 6 to 8 in optoelectrical units.

**Revendications**

**1.** Procédé de préparation d'un dérivé de fullerène à pont méthano, caractérisé en ce qu'on fait réagir un fullerène ou un dérivé de fullerène dans un solvant organique inerte, à une température de -78 à 180°C, avec un phosphorylide.

**2.** Procédé selon la revendication 1, caractérisé en ce qu'on utilise un fullerène de formule générale $C_{20+2m}$, avec m = 2... 100.

**3.** Procédé selon la revendication 1 et/ou 2, caractérisé en ce qu'on utilise un ylide de formule générale (I)

$$R^3_3\overset{\oplus}{P}-\overset{\ominus}{C}R^1R^2 \quad (I)$$

dans laquelle :

$R^1$, $R^2$ sont chacun indépendamment de l'autre H ou un groupe alkyle en $C_2$-$C_{20}$ à chaîne droite ou ramifiée (avec ou sans atome de carbone asymétrique), où un ou plusieurs groupes CH2 peuvent être remplacés par -C≡C-, -CH=CH-, -O-, -S-, -COO-, -OOC-, -$SiR^3_2$-, -CO-, ou par des groupes phénylènediyle ou cyclohexylènediyle, et où un ou plusieurs atomes d'hydrogène peuvent être remplacés par F, Cl, Br, I ou un groupe tétrahydropyrannyloxy, ou encore $R^1$ et $R^2$ forment ensemble =C=X, avec X = O, S, $NR^3$, $(-O-R^3)_2$ ;
les radicaux $R^3$ sont identiques ou différents et représentent chacun le groupe phényle ou un radical alkyle en $C_1$-$C_{12}$.

**4.** Procédé selon l'une ou plusieurs des revendications 1 à 3, caractérisé en ce que le rapport stoechiométrique de l'ylide au fullerène est de 8:1 à 0,5:1.

**5.** Procédé selon l'une ou plusieurs des revendications 1 à 4, caractérisé en ce qu'on utilise un solvant inerte du groupe comprenant le benzène, le toluène, le chlorobenzène et le chlorure de méthylène.

**6.** Dérivés de fullerènes de formule (II)

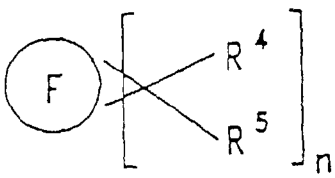

(II)

dans laquelle les symboles et indices ont les significations suivantes :

F est un résidu de fullerène de formule $C_{20+2m}$, où m vaut de 2 à 100 ;
$R^4$ est H, $CH_3$, le radical phényle ou $R^5$ ;
$R^5$ est groupe alkyle en $C_2$-$C_{20}$ à chaîne droite ou ramifiée (avec ou sans atome de carbone asymétrique), où un ou plusieurs groupes $CH_2$ peuvent être remplacés par -C≡C-, -CH=CH-, -O-, -S-, -COO-, -OOC-, -$SiR^3_2$-, -CO-, un groupe phénylènediyle ou cyclohexylènediyle, et où un ou plusieurs atomes d'hydrogène peuvent être remplacés par F, Cl, Br, I, OH ou par un groupe tétrahydropyrannyloxy, ou encore $R^4$ et $R^5$ forment ensemble =C=X, avec X = O, S, $NR^3$, $(-O-R^3)_2$ ;
$R^3$ est le radical phényle ou un groupe alkyle en $C_1$-$C_{12}$ ;
n vaut de 1 à 6.

**7.** Dérivé de fullerène selon la revendication 6, caractérisé en ce que les symboles et indices, dans la formule générale (II), ont les significations suivantes :

F est $C_{60}$, $C_{70}$ ;
$R^4$ est H ou le radical phényle ;
$R^5$ est groupe alkyle en $C_2$-$C_{20}$ à chaîne droite ou ramifiée (avec ou sans atome de carbone asymétrique), où un ou plusieurs groupes $CH_2$ peuvent être remplacés par -C≡C-, -CH=CH-, -O-, -S-, -COO-, -OOC-, -$SiR^3_2$-, -CO-, un groupe phénylènediyle ou cyclohexylènediyle, et où un ou plusieurs atomes d'hydrogène peuvent être remplacés par F, Cl, Br, I, OH ou par un groupe tétrahydropyrannyloxy, ou encore $R^4$ et $R^5$ forment ensemble =C=X, avec X = O, S, $NR^3$, $(-O-R^3)_2$ ;
$R^3$ est le radical phényle ou un groupe alkyle en $C_1$-$C_{12}$ ;
n vaut 1.

**8.** Dérivé de fullerène selon la revendication 6 et/ou 7, caractérisé en ce que les symboles et indices, dans la formule générale (II), ont les significations suivantes :

F est $C_{60}$, $C_{70}$ ;
$R^4$ est H ou le radical phényle ;
$R^5$ est groupe alkyle en $C_2$-$C_{20}$ à chaîne droite ou ramifiée où un ou plusieurs groupes $CH_2$ peuvent être remplacés par -C≡C-, -CH=CH-, -O-, -S-, -COO-, -OOC-, -$SiR^3_2$-, -CO-, un groupe phénylènediyle ou cyclohexylènediyle, et où un atome d'hydrogène peut être remplacé par OH ou par un groupe tétrahydropyrannyloxy ;
n vaut 1.

**9.** Utilisation de dérivés de fullerènes à pont méthano, préparés par un procédé selon l'une ou plusieurs des revendications 1 à 5, dans des composants optoélectriques.

**10.** Utilisation de dérivés de fullerènes de formule générale (II) selon l'une ou plusieurs des revendications 6 à 8, dans des composants optoélectriques.